# EUROPÄISCHE PATENTSCHRIFT

(11) **EP 3 151 810 B1**
(45) Veröffentlichungstag und Bekanntmachung des Hinweises auf die Patenterteilung: **08.09.2021**
(21) Anmeldenummer: 15720737.4
(22) Anmeldetag: 11.05.2015
(51) Int. Cl.: A61K 8/73, A61K 8/891, A61K 8/892, A61Q 5/02, A61Q 5/12

(54) **KONDITIONIERENDE HAARZUBEREITUNG, INSBESONDERE SHAMPOOS, MIT EINEM GEHALT AN SILICONEN UND QUATERNISIERTEN GUARDERIVATEN**
CONDITIONING HAIR PREPARATION, IN PARTICULAR SHAMPOO, CONTAINING SILICONS AND QUARTERNISED GUAR DERIVATIVES
PRÉPARATION CAPILLAIRE DE CONDITIONNEMENT, NOTAMMENT SHAMPOOINGS CONTENANT DES SILICONES ET DES DÉRIVÉS DE GUAR QUATERNISÉS

(30) Priorität: 04.06.2014 DE 102014210584
(43) Veröffentlichungstag der Anmeldung: 12.04.2017
(73) Patentinhaber: Beiersdorf AG, 20253 Hamburg (DE)
(72) Erfinder: NEMNICH, Julia, 22589 Hamburg (DE); KLAUCK, Robert, 21465 Reinbek (DE); SORS, Nathalie, 22307 Hamburg (DE)
(74) Vertreter: Beiersdorf AG
(86) Internationale Anmeldenummer: PCT/EP2015/060301
(87) Internationale Veröffentlichungsnummer: WO 2015/185330

(56) Entgegenhaltungen:
- EP-A1- 0 529 883
- EP-A2- 2 138 160
- WO-A1-2013/011122
- WO-A2-2011/107713
- DE-A1-102012 201 861
- US-A- 5 085 857
- None

## Beschreibung

Die vorliegende Erfindung betrifft eine kosmetische Zubereitung enthaltend ein oder mehrere Silicone sowie ein oder mehrere quaternisierte Guarderivate, insbesondere Guar-Hydroxypropyltrimethylammoniumchloride, ferner ein oder mehrere Tenside sowie die Verwendung dieser Zubereitung.

Bei der Körperpflege des Menschen spielt die Haarwäsche eine zentrale Rolle. Die Reinigung der Haare und der Kopfhaut von körpereigenem Fett, Hautabschilferungen, Schmutz und Gerüchen entspricht einem Grundbedürfnis des Menschen.

Zur Befriedigung dieses Bedürfnisses stand dem Menschen bis ins 20. Jahrhundert hinein allein Seife zur Verfügung, die aufgrund ihres alkalischen pH-Wertes für die Kopfhaut und die Augenschleihäute wenig verträglich war und häufig Ablagerungen von Kalkseifen im Haar zurück ließ. In den dreißiger Jahren des 20. Jahrhunderts erblickte das erste alkylsulfathaltige Shampoo das Licht der Welt. Seit Mitte der sechziger Jahre erobern Alkylethersulfate und andere Tenside den Shampoomarkt. Mit ihnen können die Nachteile von seifenhaltigen Zubereitungen vermieden werden. Heutzutage müssen moderne Shampoos das Haar nicht nur reinigen und gut verträglich sein. Vielmehr sollen sie das Haar auch pflegen und seine Frisierbarkeit und optische Attraktivität erhöhen.

Haarshampoos enthalten eine Vielzahl unterschiedlicher Komponenten um den einzelnen Anforderungen an das Produkt gerecht zu werden:
Die Reinigungskraft der Shampoos wird bewirkt durch die Anwesenheit von anionischen, amphoteren und nichtionischen Tensiden als oberflächenaktive Verbindungen in den Zubereitungen. Tenside sorgen darüber hinaus für das Schaumvermögen der Haarreinigungsmittel. Wichtig bei der Auswahl der Tenside ist darüber hinaus ihre Unempfindlichkeit gegenüber der Wasserhärte, ihre biologische Abbaubarkeit, ihre Verträglichkeit mit anderen Komponenten der Zubereitung sowie ihr Preis. Ein viel verwendetes Shampootensid ist beispielsweise Alkylethersulfat.

Darüber hinaus enthalten Shampoos eine Reihe von Konsistenzregulatoren, die der Zubereitung die gewünschte Viskosität verleihen. Diese Verdicker bewirken eine Vergrößerung der Tensidmicellen bzw. eine Quellung der Wasserphase der Zubereitung. Verdicker können aus chemisch sehr unterschiedlichen Stoffklassen gewählt werden. So werden u.a. Elektrolyte (z.B. Natriumchlorid), Alkanolamide (z.B. Fettsäure-Monoethanolamide), niedrig ethoxylierte Fettalkohole (z.B. Diethylenglycol-monolaurylether), hochethoxylierte Ether, Ester und Diester sowie polymere Verdicker eingesetzt. Zu den polymeren Verdickern zählen beispielsweise Celluloseether. Darüber hinaus finden auch Polyacrylate und Hydrokolloide als Verdicker Verwendung. Polymere Verdicker haben den großen Vorteil, dass die durch sie erzeugte Viskosität weitgehend temperaturunabhängig ist.

Neben Parfüm- und Farbstoffen sowie einer Reihe von Verbindungen, welche die Haltbarkeit der Zubereitungen erhöhen, werden in jüngerer Zeit den Haarshampoos unterschiedliche Arten von Wirkstoffen zugefügt. Hierzu zählen neben UV-Absorbern, Vitaminen oder Pflanzenextrakten auch sogenannte Haarkonditionierer (engl. conditioner), welche das Haar pflegen und seine Kämmbarkeit und seinen Griff verbessern sowie seinen Glanz erhöhen. Konditionierer ziehen, im Gegensatz zu den meisten anderen Bestandteilen von Shampoos, auf das Haar auf und verbleiben dort nach dem Spülen. Sie lagern sich aufgrund ihres Molekülaufbaus an die Schadstellen der Cuticula des Haares und glätten das Haar. Dadurch wird das Haar weniger rauh und spröde, die Frisur bekommt deutlich mehr Glanz und lässt sich leichter kämmen. Auch wird das Haar weniger empfindlich für eine elektrostatische Aufladung. Die wichtigsten haarkonditionierenden Substanzen in Shampoos stellen die polymeren quatären Ammoniumverbindungen dar. Auch können kationische Cellulosederivate und Polysaccharide eingesetzt werden. Weiterhin werden auch Silikonverbindungen zur Konditionierung eingesetzt.

Nachteilig am Stande der Technik ist der Umstand, dass es bisher nur unzureichend gelungen ist, die Menge des sich auf dem Haar ablagernden Konditionierers zu regulieren. Meist kommt es bei der wiederholten Anwendung von Haarkonditioniern zu einer Zunahme der Menge an Konditionierer auf dem Haar. Durch die Zunahme von Konditionierern auf dem Haar wird dieses mehr und mehr beschwert (engl. build up-effect), die Frisur erscheint ungepflegt und strähnig. Dies ist insbesondere bei längeren Haaren der Fall, die der Pflege durch Konditionierer in besonderem Maße bedürfer Die Patentdokumente US 5085857 und EP 0529883 betreffen beide Konditionierungsshampoo-Zusammensetzungen.

Es war daher die Aufgabe der vorliegenden Erfindung eine kosmetische Zubereitung und insbesondere ein Haarreinigungsmittel (Shampoo) zu entwickeln, welches die Mängel des Standes der Technik beseitigt oder zumindest lindert.

Überraschend gelöst wird die Aufgabe durch kosmetische Zubereitungen, insbesondere Shampoos, welche umfassen
a) eine wässrige Phase, welche eine oder mehrere grenz- bzw. oberflächenaktive Substanz oder Substanzen enthält,
b) ein oder mehrere Guar-Hydroxypropyltrimethylammoniumchloride, welches oder welche aus der Gruppe der Derivate gewählt wird oder werden, das oder die Ladungsdichten von 0,4 bis 1,0 meq/g , bevorzugt 0,6 bis 0,8 meq/g, sowie ein Molekulargewicht von 1000000 bis 3000000 g/mol, bevorzugt 2000000 bis 3000000 g/mol, aufweist oder aufweisen,
c) ein oder mehrere Silikonölemulsionen, gewählt aus der Gruppe der Dimethiconol-Emulsionspolymerisationstypen mit Viskositäten von 800000 bis 1300000 mPas, bevorzugt 800000 mPas, sowie eine Tröpfchengröße von 200 bis 350 nm, bevorzugt 200 nm, einer Konzentration von 1 bis 2 Gewichts-% entsprechend, bezogen auf das Gesamtgewicht der Zubereitung.

Die erfindungsgemäßen Zubereitungen führen zu einer weit verbesserten Nasskämmbarkeit sowie zu einer deutlich verbesserten Silikondeposition auf verschiedenen Haarfaserqualitäten als es bei herkömmlichen Mischungen von Silikonshampoos mit kationischen Polymeren der Fall ist. Selbst langes Haar wird auch bei wiederholter Anwendung der erfindungsgemäßen Zubereitung kaum beschwert und ist von seidigem Glanz.

Erfindungsgemäß vorteilhaft sind insbesondere Guar-Derivate der Serie Jaguar. Erfindungsgemäß ganz besonders bevorzugt ist das Guar-Derivat Jaguar C14-S der Firma Rhodia.

| | |
|---|---|
| Jaguar C14-S | Guar Hydroxypropyltrimonium Chloride |
| Jaguar C-162 | Guar Hydroxypropyltrimonium Chloride |
| Jaguar Excel | Guar Hydroxypropyltrimonium Chloride |

Die kosmetischen Zubereitungen im Sinne der Erfindung enthalten vorteilhafter Weise ein oder mehrere Guar-Hydroxypropyltrimethylammoniumchloride in einer Konzentration von 0,01 bis 10 Gewichts-%, bevorzugt in einer Konzentration von 0,01 bis 2 Gewichts- und ganz besonders bevorzugt in einer Konzentration von 0,1 bis 0,2 Gewichts-%, jeweils bezogen auf das Gesamtgewicht der Zubereitung.

Heutzutage werden Silikonölemulsionen angeboten, bei denen durch den Emulsionsprozess hochmolekulare Silikone in einer flüssigen Form dargereicht und einfacher verarbeitet werden können. Dimethiconol-Emulsionspolymerisate stellen Öl/Wasser-Emulsionen dar, die neben dem Dimethiconol noch Wasser, einen Emulgator - der gelichzeitig auch als saurer Katalysator agiert - wie Natriumdodecylbenzolsulfonat, ein Konservierungsmittel und eine Base wie Triethanolamin oder Natriumlauge enthalten.

So können zielgerichtet bei der Emulsinspolymerisation Molekulargewicht sowie Tröpfchengröße der Silikontröpfchen eingestellt werden. Im Emulsionspolymerisationsprozess wird das Dimethiconol geildet. Die Viskosität des Dimethiconoles und die Partikelgröße können über spezielle Optionen eingestellt werden. Ebenso können Dimethicone-Emulsionen produziert werden. Hier wird das Dimethicone final emulgiert. Bei Dimethicone-Emulsionen existieren Limits bzgl. der minimal möglichen Tröpfchengröße sowie der maximal möglichen Viskosität.

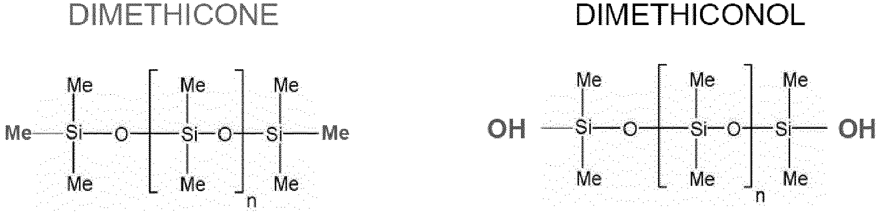

Erfindungsgemäß ganz besonders bevorzugt ist das Silsoft EM-160A der Firma Momentive.

| **Handelsname** | **INCI** |
|---|---|
| Silsoft EM-160A | Dimethiconol |
| Wacker Belsil 6010 | Dimethiconol |

Die kosmetischen Zubereitungen im Sinne der Erfindung enthalten vorteilhafter Dimethiconol-Emulsionen in einer Konzentration von 1 bis 2 Gewichts-%, jeweils bezogen auf das Gesamtgewicht der Zubereitung.

Beispiele für die im Rahmen der vorliegenden Erfindung verwendbaren kationischen Tenside sind insbesondere quartäre Ammoniumverbindungen. Bevorzugt sind Antmoniumhalogenide wie Alkyltrimethylammoniumchloride, Dialkyldimethylammoniumchloride und Trialkylmethylammoniumchloride, z. B. Cetyltrimethyl-ammoniumchlorid, Stearyltrimethylammoniumchlorid, Distearyldimethylammonium-chlorid, Lauryldimethylammoniumchlorid, Lauryldimethylbenzylammoniumchlorid und Tricetylmethylammoniumchlorid. Alkylamidoamine, insbesondere Fettsäureamidoamine wie das unter der Bezeichnung Tego Amid TM S 18 erhältliche Stearylamidopropyldimethylamin, zeichnen sich weiterhin durch ihre gute biologische Abbaubarkeit aus. Ebenfalls sehr gut biologisch abbaubar sind quaternäre Esterverbindungen, sogenannte "Esterquats", wie die unter dem Warenzeichen Stepantex TM vertriebenen Methylhydroxyalkyl-dialkoyloxyalkyl-ammonium-methosulfate sowie die entsprechenden Produkte, die unter dem Warenzeichen Dehyquart TM im Handel erhältlich sind. Ein Beispiel für ein als kationisches Tensid einsetzbares quaternäres Zuckerderivat stellt das Handelsprodukt Glucquat TM 100 dar, gemäß CTFA-Nomenklatur ein "Lauryl Methyl Gluceth-10 Hydroxypropyl Dimonium Chloride".

Als anionische Tenside eignen sich alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ether-, Amid- und Hydroxylgruppen sowie in der Regel auch Estergruppen enthalten sein. Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C8-C22-Carbonsäuren, wie Ölsäure, Stearinsäure, Isostearinsäure und Palmitinsäure. Besonders bevorzugt sind die anionischen Tenside, die mindestens eine Carboxylat-Gruppe enthalten.

Erfindungsgemäße Zubereitungen enthalten mindestens eine oder mehrere grenz- bzw. oberflächenaktive Substanz oder Substanzen, landläufig als Tenside bezeichnet, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Anionische Tenside können dabei ganz besonders bevorzugt sein.

Bezüglich der anionischen und kationischen Tenside wird auf das oben gesagte verwiesen.

Als zwitterionische Tenside werden solche oberflächenaktiven Verbindungen bezeichnet, die im Molekül mindestens eine quartäre Ammoniumgruppe und mindestens eine -COO(<->)- oder -SO3(<->)-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethyl-ammoniumglycinate, beispielsweise das Kokosacylamino-propyl-dimethylammonium-glycinat, und 2-Alkyl-3-carboxymethyl-3-hydroxyethyl-imidazoline mit jeweils 8 bis 18 C-Atomen in der Alkyl- oder Acylgruppe sowie das Kokosacyl-aminoethyl-hydroxyethylcarboxymethylglycinat. Ein bevorzugtes zwitter-ionisches Tensid ist das unter der CTFA-Bezeichnung Cocamidopropyl Betaine bekannte Fettsäureamid- Derivat.

Unter ampholytischen Tensiden werden solche oberflächenaktiven Verbindungen verstanden, die außer einer C8-C18-Alkyl- oder -Acylgruppe im Molekül mindestens eine freie Aminogruppe und mindestens eine -COOH- oder -SO3H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C12-18-Acylsarcosin.

Nichtionische Tenside enthalten als hydrophile Gruppe z. B. eine Polyolgruppe, eine Polyalkylenglykolethergruppe oder eine Kombination aus Polyol- und Polyglykol-ethergruppe. Solche Verbindungen sind beispielsweise:
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C12-C22-Fettsäuremono- und -diester von Anlagerungsprodukten von 1 bis 30 Mol Ethylenoxid an Glycerin,
- C8-C22-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester,
- Anlagerungsprodukte von Ethylenoxid an Fettsäurealkanolamide.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyltrimethylammoniumchlorid/Acrylat-Copolymere und Octylacrylamid/Methylmethacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert. Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, CelluloseDerivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Maleinsäure und Milchsäure,
- haarkonditionierende Verbindungen wie Phospholipide, beispielsweise Sojalecithin, EiLecithin und Kephaline, sowie Silikonöle,
- Proteinhydrolysate, insbesondere Elastin-, Kollagen-, Keratin-, Milcheiweiss-, Sojaprotein- und Weizenproteinhydrolysate, deren Kondensationsprodukte mit Fettsäuren sowie quaternisierte Proteinhydrolysate,
- Dimethylisosorbid und Cyclodextrine,
- Lösungsmittel und -vermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes, wie beispielsweise alpha - und beta -Hydroxycarbonsäuren
- Wirkstoffe wie Panthenol, Pantothensäure, Allantoin, Pyrrolidoncarbonsäuren und deren Salze, Pflanzenextrakte und Vitamine,
- Cholesterin,
- Konsistenzgeber wie Zuckerester, Polyolester oder Polyolalkylether,
- Fette und Wachse wie Walrat, Bienenwachs und Montanwachs,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. K. Schrader, Grundlagen und Rezepturen der Kosmetika, 2. Auflage, Hüthig Buch Verlag, Heidelberg, 1989, verwiesen.

Die folgenden Beispiele sollen den Erfindungsgegenstand weiter erläutern.

**Beispielrezepturen:**

| | | **Shampoo 1** | **Shampoo 2** | **Shampoo 3** | **Shampoo 4** |
|---|---|---|---|---|---|
| | | | | | |
| 1 | Natriumlaurethsulfat | 11 | 12 | 13 | 11 |
| 2 | Cocamidopropylbetain | 1 | 2 | 1 | 1,2 |
| 3 | Cocamid MEA | 0 | 0 | 0 | 1 |
| 4 | Cocamid DEA | 0 | 0 | 0 | 1,5 |
| 5 | Natrium Cocoamphoacetat | 0 | 0 | 0 | 1 |
| 6 | Polyquaternium-10 | 0 | 0 | 0 | 0,3 |
| 7 | Guar Hydroxypropyltrimonium Chlorid | 0,1 | 0,2 | 0,3 | 0,2 |
| 8 | Polyquaternium-7 | 0 | 0 | 0 | 2 |
| 9 | Dimethiconol | 1 | 2 | 1,5 | 2 |
| 10 | Trimethylsiloxysilicat, Propylsilsesquioxane | 0 | 0 | 0 | 0,05 |
| 11 | Dimethicone(DM5 - 5mm2/s Visc.) | 0 | 0 | 0 | 0,05 |
| 12 | PEG-3 Distearat | 1,5 | 0 | 1 | 1,8 |
| 13 | Glycoldistearat | 0 | 0 | 0 | 1,5 |
| 14 | PEG-90 M | 0 | 0 | 0 | 0,025 |
| 15 | Natriumbenzoat | 0,56 | 0,56 | 0,4 | 0,6 |
| 16 | NaOH | 0,01 | 0 | 0,03 | 0 |
| 17 | Methylisothizolinon | 0 | 0 | 0 | 0,2 |
| 18 | Phenoxyethanol | 0 | 0 | 0 | 0,9 |
| 19 | Benzophenone-4 | 0 | 0 | 0 | 0,5 |
| 20 | PEG-40 Hydriertes Rizinusöl | 0 | 0 | 0 | 0,4 |
| 21 | Parfüm | 0,6 | 0,5 | 0,5 | 0,6 |
| 22 | Citronensäure | Ad pH 5,2 | Ad pH 5,2 | Ad pH 5,0 | Ad pH 5,0 |
| 23 | NaCl | Ad Visc 5000 mPas | Ad Visc 6000 mPas | Ad Visc. 5000 mPas | Ad Visc. 6000 mPas |
| 24 | Sodium Salicylate | 0,2 | 0,2 | 0,2 | |
| | Wasser | Ad 100 | Ad 100 | Ad 100 | Ad 100 |

### Handelsnamen:

- 1.: Ethersulfat K25 NK, Beiersdorf
- 2.: Tego Betain F 50, Evonik Goldschmidt
- 3.: Comperlan 100, BASF
- 4.: Comperlan KD, BASF
- 5.: Rewoteric AM C, Evonik
- 6.: Ucare Polymer JR-400, Dow Chemical
- 7.: Jaguar-Typen, Solvay
- 8.: Merquat 550, Lubrizol
- 9.: Silsoft EM-160 A, Momentive
- 10.: Dow Corning MQ-1640 Flake Resin, Dow Corning
- 11.: BRB DM 5, BRB International
- 12.: PEG-3 Distearate Solution, Beiersdorf
- 13.: Euperlan PK 300 OK, BASF
- 14.: Polyox WSR-301, Nordmann & Rassmann
- 15.: Natriumbenzoat EDF BP 98, Wuhan Youji Industries
- 16.: Natronlauge 45%, Brenntag
- 17.: Neolone 950, Dow Chemical
- 18.: Phenoxyethanol R +, BASF
- 19.: Natronlauge 45%, 3V Sigma
- 20.: Eumulgin CO 40, BASF
- 21.: /
- 22.: Citronensäuremonohydrat, Jungbunzlauer
- 23.: Suprasel fine, Akzo Nobel
- 24.: Natriumsalicylat BP 93, Schütz & Co

### Experimentelle Beispiele:

Die erfindungsgemäßen Zubereitungen führen zu einer weit verbesserten Nasskämmbarkeit sowie zu einer deutlich verbesserten Silikondeposition auf verschiedenen Haarfaserqualitäten als es bei herkömmlichen Mischungen von Silikonshampoos mit kationischen Polymeren der Fall ist. Selbst langes Haar wird auch bei wiederholter Anwendung der erfindungsgemäßen Zubereitung kaum beschwert und ist von seidigem Glanz.

### Messung der Silikondeposition mittels IR-Spektroskopie:

Die Deposition von Silikon auf Haaren wurde mit Hilfe der Fourier-Transformation-IR und einem ATR-Germaniumkristall ermittelt. Es wurde das FT-IR -Gerät Vector 22 mit ATR-Einheit der Firma Bruker eingesetzt. Pro Formulierung werden 2 Haartressen an jeweils 5 verschiedenen Punkten vermessen, wobei die oberen 2 und untern 5 cm ausgelassen werden. Die Auswertung erfolgt durch die Integration der Silikonbande (1276,6 bis 1245,9 cm⁻¹) und der 2. Amidbande (1585 bis 1486,9 cm⁻¹). Das Verhältnis der beiden Flächen, von Silikon zu Protein, wird gebildet.

### Probenvorbereitung - Behandlung der zu untersuchenden Tressen:

Für die Probenvorbereitung werden Handschuhe getragen, diese werden vor der Applikation mit dem zu untersuchenden Produkten gewaschen. Die Haartresse wird zunächst gründlich gereinigt und anschließend zweimal mit dem Produkt gewaschen. Die Tresse wird über Nacht an der Luft getrocknet. Alle Tests werden mit Verschnittwasser (konstante Wasserhärte und Temperatur) durchgeführt. Für die geschädigten Haare werden Haartressen im Labor selber moderat mit Wasserstoffperoxide geschädigt.

| | ungeschädigt | geschädigt |
|---|---|---|
| Elvital Color Glanz | 0,0190 | 0,0061 |
| 0,1% Jaguar C14 S, 1% Silsoft | 0,0578 | 0,0356 |
| 0,1% Jaguar C14 S, 1,5% Silsoft | 0,0893 | 0,0309 |
| 0,2% Jaguar C14 S, 1% Silsoft | 0,0632 | 0,0355 |
| 0,2% Jaguar C14 S, 1,5% Silsoft | 0,1030 | 0,0479 |
| Elvital Color Glanz | 0,0190 | 0,0076 |
| 0,1% Jaguar C14 S, 1% MEM-1664 | 0,0481 | 0,0286 |
| 0,1% Jaguar C14 S, 1,5% MEM-1664 | 0,0685 | 0,0287 |
| 0,2% Jaguar C14 S, 1% MEM-1664 | 0,0442 | 0,0165 |
| 0,2% Jaguar C14 S, 1,5% MEM-1664 | 0,0422 | 0,0297 |

Diese Abbildung zeigt, dass Silsoft EM-160A im Vergleich zu einem anderen Silikon auch in Kombination mit Jaguar C14 S und dem Marktprodukt die beste Silikondeposition auf ungeschädigten Haaren zeigt.

### Durchführung der Kämmkraftmessung:

Für die Durchführung des sogenannten rinse-profile werden ungeschädigte Haare verwendet. Es wird die Nasskämmbarkeit vor und nach der Behandlung mit dem zu untersuchenden Produkt zu unterschiedlichen Zeitpunkten während des Ausspülens ermittelt (siehe Abbildung 3):
- Nach 15 Sekunden spülen
- Nach 30 Sekunden spülen
- Nach 60 Sekunden spülen
- Nach 90 Sekunden spülen

In den folgenden Tabellen ist zu sehen, dass die Formeln mit Silsoft EM-160 A die Kämmkräfte signifikant reduzieren und allgemein auf einem geringeren Niveau als die Formel mit dem anderen Silikon und als das Marktprodukt liegen.

### 5 Results

### 5.1 Mean Combing Force

### 5.1.1 Descriptive Statistics

**Table 1a: Descriptive statistics of original data for mean combing force**

| **KKN_MeanForce** | | | | | | |
|---|---|---|---|---|---|---|
| **Product** | **Descriptive Statistics** | **Original data** | | | | |
| | | **untreated** | **treated, 15s rinsing** | **treated, 30s rinsing** | **treated, 60s rinsing** | **treated, 90s rinsing** |
| 10 | N | 7 | 7 | 7 | 7 | 7 |
| | mean | 302.47 | 117.81 | 119.43 | 123.00 | 131.89 |
| | standard deviation | 11.87 | 2.27 | 4.57 | 4.57 3.61 | 9.92 |
| | median | 303.21 | 118.58 | 119.28 | 122.12 | 131.81 |
| | IQR | 23.84 | 2.55 | 385 | 6.55 | 7.15 |
| 20 | N | 7 | 7 | | 7 | 7 |
| | mean | 314.28 | 78.25 | 71.95 | 74.45 | 75.46 |
| | Standard deviation | 13.17 | 4.88 | 4.86 | 6.76 | 3.37 |
| | median | 311.37 | 78.11 | 71.56 | 74.53 | 77.39 |
| | IQR | 16.60 | 5.36 | 7.70 | 5.26 | 6.89 |
| 30 | N | 7 | 7 | | 7 | 7 |
| | mean | 316.75 | 133.99 | 139.32 | 156.33 | 165.00 |
| | Standard deviation | 16.60 | 7.23 | 8.21 | 12.30 | 11.23 |
| | median | 316.16 | 133.95 | 135.56 | 152.47 | 164.13 |
| | IQR | 22.34 | 15.24 | 11.83 | 21.76 | 19.13 |
| 40 | N | 7 | 7 | 7 | 7 | 7 |
| | mean | 313.18 | 74.99 | 70.15 | 74.09 | 76.60 ' |
| | standard deviation | 8.29 | 4.90 | 5.59 | 3.79 | 5.69 |
| | median | 316.32 | 74.53 | 69.00 | 73.96 | 75.32 |
| | IQR | 8.75 | 6.08 | 11.85 | 3.54 | 10.70 |
| 50 | N | 7 | 7 | 7 | 7 | 7 |
| | mean | 311.67 | 130.59 | 136.40 | 152.12 | 156.34 |
| | Standard deviation | 13.39 | 9.67 | 10.91 | 1 11.99 | 5.44 |
| | median | 308.40 | 129.57 | 131.49 | 153.25 | 158.27 |
| | IQR | 27.00 | 3.42 | 16.25 | 23.09 | 9.16 |

## Patentansprüche

1. Kosmetische Zubereitungen, insbesondere Shampoos, welche umfassen
a) eine wässrige Phase, welche eine oder mehrere grenz- bzw. oberflächenaktive Substanz oder Substanzen enthält,
b) ein oder mehrere Guar-Hydroxypropyltrimethylammoniumchloride, welches oder welche aus der Gruppe der Derivate gewählt wird oder werden, das oder die Ladungsdichten von 0,4 bis 1,0 meq/g , bevorzugt 0,6 bis 0,8 meq/g, sowie ein Molekulargewicht von 1000000 bis 3000000 g/mol, bevorzugt 2000000 bis 3000000 g/mol, aufweist oder aufweisen,
c) eine oder mehrere Silikonölemulsionen, gewählt aus der Gruppe der Dimethiconol-Emulsionspolymerisationstypen mit Viskositäten von 800000 bis 1300000 mPas, bevorzugt 800000 mPas, sowie eine Tröpfchengröße von 200 bis 350 nm, bevorzugt 200 nm, einer Konzentration von 1 bis 2 Gewichts-% entsprechend, bezogen auf das Gesamtgewicht der Zubereitung.

2. Zubereitungen nach einem der vorstehenden Ansprüche, **dadurch gekennzeichnet, dass** das oder die Guar-Hydroxypropyltrimethylammoniumchloride in einer Konzentration von 0,01 bis 10 Gewichts-%, bevorzugt in einer Konzentration von 0,01 bis 2 Gewichts- und ganz besonders bevorzugt in einer Konzentration von 0,1 bis 0,2 Gewichts-%, vorliegt oder vorliegen, jeweils bezogen auf das Gesamtgewicht der Zubereitung.

## Claims

1. Cosmetic preparations, especially shampoos, which comprise
a) an aqueous phase comprising one or more boundary- or surface-active substance or substances,
b) one or more guar hydroxypropyltrimethylammonium chlorides which is or are selected from the group of derivatives which has or have charge densities of 0.4 to 1.0 meq/g, preferably 0.6 to 0.8 meq/g, and has or have a molecular weight of 1 000 000 to 3 000 000 g/mol, preferably 2 000 000 to 3 000 000 g/mol,
c) one or more silicone oil emulsions selected from the group of dimethiconol emulsion polymerization types having viscosities of 800 000 to 1 300 000 mPas, preferably 800 000 mPas, and a droplet size of 200 to 350 nm, preferably 200 nm, corresponding to a concentration of 1 to 2% by weight, based on the total weight of the preparation.

2. Preparations according to any of the preceding claims, **characterized in that** the guar hydroxypropyltrimethylammonium chloride(s) is or are present at a concentration of 0.01 to 10% by weight, preferably at a concentration of 0.01 to 2% by weight and especially preferably at a concentration of 0.1 to 0.2% by weight, based in each case on the total weight of the preparation.

## Revendications

1. Préparations cosmétiques, en particulier shampooings, lesquelles comprennent
a) une phase aqueuse, laquelle contient une ou plusieurs substance(s) tensioactive(s) ou active(s) en surface,
b) un ou plusieurs chlorures de guar-hydroxypropyltriméthylammonium choisi(s) dans le groupe des dérivés qui présente(nt) la ou les densité(s) de charge de 0,4 à 1,0 méq/g, préférablement 0,6 à 0,8 méq/g, ainsi qu'un poids moléculaire de 1 000 000 à 3 000 000 g/mole, préférablement 2 000 000 à 3 000 000 g/mole,
c) une ou plusieurs émulsions d'huiles de silicone, choisies dans le groupe des types de polymérisation en émulsion de diméthiconol comportant des viscosités de 800 000 à 1 300 000 mPas, préférablement 800 000 mPas, ainsi qu'une taille de gouttelette de 200 à 350 nm, préférablement de 200 nm, correspondant à une concentration de 1 à 2 % en poids, par rapport au poids total de la préparation.

2. Préparations selon l'une des revendications précédentes, **caractérisées en ce que** le ou les chlorures de guar-hydroxypropyltriméthylammonium est/sont présent(s) en une concentration de 0,01 à 10 % en poids, préférablement en une concentration de 0,01 à 2 % en poids et tout particulièrement préférablement en une concentration de 0,1 à 0,2 % en poids, à chaque fois par rapport au poids total de la préparation.
